# EUROPEAN PATENT SPECIFICATION

(11) **EP 0 966 534 B1**
(45) Date of publication and mention of the grant of the patent: **02.02.2005**
(21) Application number: 98909593.0
(22) Date of filing: 10.03.1998
(51) Int. Cl.: C12N 15/33, C12N 5/10, A61K 48/00

(54) **COMPOSITIONS AND METHODS FOR ELIMINATION OF UNWANTED CELLS**
ZUSAMMENSETZUNGEN UND VERFAHREN ZUR ELIMINIERUNG UNERWÜNSCHTER ZELLEN
COMPOSITIONS ET METHODES D'ELIMINATION DE CELLULES INDESIRABLES

(30) Priority: 11.03.1997 GB 9705007; 30.04.1997 US 45164 P
(43) Date of publication of application: 29.12.1999
(73) Proprietor: Mayo Foundation for Medical Education and Research, Rochester, MN 55906 (US)
(72) Inventor: RUSSELL, Stephen James, Cambridge CB2 5ER (GB); MORLING, Frances Joanne, Cambridge CB1 4SA (GB); FIELDING, Adele Kay, Cambridge CB4 6AW (GB); COSSET, François-Loic, F-69004 Lyon (FR); CATTANEO, Roberto, CH-8044 Zurich (CH)
(74) Representative: Nash, Keith Wilfrid
(86) International application number: PCT/GB1998/000710
(87) International publication number: WO 1998/040492

(56) References cited:
- EP-A- 0 449 116
- EP-A- 0 790 312
- WO-A-94/25600
- RUSSELL S J: "Replicating vectors for gene therapy of cancer: Risks, limitations and prospects" THE EUROPEAN JOURNAL OF CANCER, vol. 30A, no. 8, 1994, ISSN:0959-8049, pages 165-1171, XP002069966 cited in the application
- ZHANG J AND RUSSELL S J: "Vectors for cancer gene therapy" CANCER AND METASTASIS REVIEWS, vol. 15, no. 3, September 1996, ISSN:01767-7659, pages 385-401, XP002069967
- COSSET F-L AND RUSSELL S J: "Targeting retrovirus entry" GENE THERAPY, vol. 3, no. 11, November 1996, ISSN:0969-7128, pages 946-956, XP002069968 cited in the application

## Description

### Related Applications

This application claims the benefit of US provisional Application No. 60/045,164, filed 30th April 1997.

### Field of the Invention

This invention concerns the therapeutic use of genes encoding syncytium-inducing viral membrane glycoproteins for selective elimination of unwanted cells.

### Background of the Invention

### Viral membrane glycoproteins mediating cell-cell fusion

Enveloped viruses have membrane spike glycoproteins for attachment to mammalian cell surfaces and for subsequent triggering of membrane fusion, allowing for viral entry into the cell. In some viruses attachment and fusion triggering are mediated by a single viral membrane glycoprotein, but in other viruses these functions are provided by two or more separate glycoproteins. Sometimes (e.g. Myxoviridae, Togaviridae, Rhabdoviridae) the fusion triggering mechanism is activated only after the virus has entered into the target cell by endocytosis, at acid pH.

Other viruses (e.g. Paramyxoviridae, Retroviridae, Herpesviridae, Coronaviridae) can fuse directly with the target cell membrane at neutral pH and have an associated tendency to trigger membrane fusion between infected target cells and neighbouring noninfected cells. The visible outcome of this latter tendency for triggering of cell-cell fusion is the formation of cell syncytia containing up to 100 nuclei (also known as polykaryocytes or multinucleated giant cells). Syncytium-formation results in the death of the cells which make up the syncytium. Viral membrane proteins of these latter groups of viruses are of particular interest in the present invention.

Viruses of the Family Paramyxoviridae have a strong tendency for syncytium induction which is dependent in most cases on the co-expression of two homo-oligomeric viral membrane glycoproteins, the fusion protein (F) and the viral attachment protein (H, HN or G). Co-expression of these paired membrane glycoproteins in cultured cell lines is required for syncytium induction although there are exceptions to this rule such as SV5 whose F protein alone is sufficient for syncytium induction. F proteins are synthesised initially as polyprotein precursors (Fₒ) which cannot trigger membrane fusion until they have undergone a cleavage activation. The activating protease cleaves the Fₒ precursor into an extraviral F₁ domain and a membrane anchored F₂ domain which remain covalently associated through disulphide linkage. The activating protease is usually a serine protease and cleavage activation is usually mediated by an intracellular protease in the Golgi compartment during protein transport to the cell surface. Alternatively, where the cleavage signal is not recognised by a Golgi protease, the cleavage activation can be mediated after virus budding has occurred, by a secreted protease (e.g. trypsin or plasmin) in an extracellular location (Ward *et al*, Virology, 1995, 209, p242-249; Paterson *et al*, J. Virol., 1989, 63, 1293-1301).

WO 94/25600 is concerned with and discloses recombinant antigens from Mumps virus and their use in vaccines. In particular, the document discloses an experiment in which recombinant vaccinia viruses were used to inoculate hamsters, the recombinant viruses expressing a single Mumps virus polypeptide, such as F protein or HN protein. Since these proteins are not fusogenic in isolation, there is no disclosure of a composition comprising a vector expressing a fusogenic combination of Mumps virus polypeptides.

Certain members of the Herpesvirdae family are renowned for their potent syncytium-inducing activity. Indeed, Varicella-Zoster Virus has no natural cell-free state in tissue culture and spreads almost exclusively by inducing cell fusion, forming large syncytia which eventually encompass the entire monolayer. gH is a strongly fusogenic glycoprotein which is highly conserved among the herpesviruses. Maturation and membrane expression of gH are dependent on coexpression of the virally encoded chaperone protein gL (Duus *et al*, Virology, 1995, 210, 429-440). Although gH is not the only fusogenic membrane glycoprotein encoded in the herpesvirus genome (gB has also been shown to induce syncytium formation), it is required for the expression of virus infectivity (Forrester *et al*, J. Virol., 1992, 66, 341-348)

Retroviruses use a single homooligomeric membrane glycoprotein for attachment and fusion triggering. Each subunit in the oligomeric complex is synthesised as a polyprotein precursor which is proteolytically cleaved into membrane-anchored (TM) and extraviral (SU) components which remain associated through covalent or noncovalent interactions.

Cleavage activation of the retroviral envelope precursor polypeptide is usually mediated by a Golgi protease during protein transport to the cell surface. There are inhibitory (R) peptides on the cytoplasmic tails of the TM subunits of the envelope glycoproteins of murine leukaemia virus (MLV) and Mason Pfizer monkey virus (MPMV) which are cleaved by the virally encoded protease after virus budding has occurred. Cleavage of the R peptide is required to activate fully the fusogenic potential of these envelope glycoproteins and mutants lacking the R peptide show greatly enhanced activity in cell fusion assays (Rein *et al*, J. Viral., 1994. 68, 1773-1781; Ragheb & Anderson, J. Viral., 1994, 68, 3220-3231; Brody *et al*, J. Virol., 1994, 68, 4620-4627).

Naturally occurring MLV strains can also differ greatly in their propensity for syncytium induction in specific cell types or tissues. One MLV variant shows a strong tendency to induce the formation of endothelial cell syncytia in cerebral blood vessels, leading to intracerebral haemorrhages and neurologic disease. The altered behaviour of this MLV variant can be reproduced by introducing a single point mutation in the env gene of a non-neurovirulent strain of Friend MLV, resulting in a tryptophan-to-glycine substitution at amino acid position 120 in the variable region of the SU glycoprotein (Park *et al*. J. Virol., 1994, 68, 7516-7524). HIV strains are also known to differ greatly in their ability to induce the formation of T cell syncytia and these differences are known to be determined in large part by variability between the envelope glycoproteins of different strains.

The membrane glycoproteins of viruses that normally trigger fusion at acid pH do not usually promote syncytium formation. However, they can trigger cell-cell fusion under certain circumstances. For example, syncytia have been observed when cells expressing influenza haemagglutinin or the G protein of Vesicular Stomatitis Virus are exposed to acid (Steinhauer *et al*, Proc. Natl. Acad. Sci. USA 1996, 93, 12873-12878) or when the fusogenic glycoproteins are expressed at a very high density (Yang *et al*, Hum. Gene Ther. 1995, 6, 1203-1213). In addition, acid-triggered fusogenic viral membrane glycoproteins can be mutated to shift their pH optimum for fusion triggering (Steinhauer *et al*, Proc. Natl. Acad. Sci. USA 1996, 93, 12873-12878).

The ability of poxviruses to cause cell fusion at neutral pH correlates strongly with a lack of HA production (Ichihashi & Dales, Virology, 1971, 46, 533-543). Wild type vaccinia virus, an HA-positive orthopoxvirus, does not cause cell fusion at neutral pH, but can be induced to do so by acid pH treatment of infected cells (Gong *et al*, Virology, 1990, 178, 81-91). In contrast, wild type rabbitpox virus, which lacks a HA gene, causes cell fusion at neutral pH. However, inactivation of the HA or SPI-3 (serpin) genes in HA-positive orthopoxviruses leads to the formation of syncytia by fusion of infected cells at neutral pH (Turner & Moyer, J. Virol. 1992, 66, 2076-2085). Current evidence indicates that the SPI-3 and HA gene products act through a common pathway to control the activity of the orthopoxvirus fusion-triggering viral glycoproteins, thereby preventing fusion of cells infected with wild type virus.

Promotion of cell-to-cell fusion by a given virus is strongly influenced by the type of cell infected (Reviewed in Poste, Adv. Virus Res. 1970, 303-356). There are many known examples in which the same virus strain can cause extensive syncytium formation of one cell type but not of another, yet replicate equally well in both cell types. Numerous host cell factors are known to play a role in determining the likelihood that a particular pair of cells will fuse with each other when one of the fusion partners is expressing a particular fusogenic membrane glycoprotein complex. Contributory factors include the expression of suitable processing proteases and membrane receptors, the lipid composition of the plasma membrane (Daya *et al*, Virology, 1988, 163, 276-283) and the thickness of the glycocalyx (Poste, Adv. Virus Res. 1970, 303-356).

In general, established transformed cell lines are more susceptible to formation of syncytia than nontransformed cell lines or primary cells. For polarised epithelial cells it has been shown that basolateral expression of (proteolytically activated) Sendai virus envelope glycoproteins is required to induce cell fusion. Wild type Sendai virus F glycoproteins are targeted to the apical domain of polarised MDCK cells and do not induce cell fusion even when the F glycoprotein is activated with trypsin. However, F protein mutants which bud bipolarly at the apical and basolateral domains are able to induce cell fusion of MDCK cells, provided the basolaterally expressed protein is in its active conformation (Tashiro *et al*, Arch. Virol. 1992, 125, 129-139).

Lack of syncytium formation does not necessarily indicate lack of fusion activity. Variant herpesviruses and paramyxoviruses exist that do not yield syncytia even though they are fusion competent. Sometimes the non-syncytial phenotype maps to the membrane glycoproteins that are implicated in fusion triggering (Cai *et al*, J. Virol. 1988, 62. 2596-2604) but in other cases, it maps to other viral genes. The density of the fusion proteins at the cell surface is also an important determinant of the likelihood of cell-cell fusion (Gething *et al*, J. Cell Biol. 1986, 102, 11-23).

### Therapeutic genes for cancer gene therapy

Attempts to treat human malignancy by gene therapy have, to date, been ineffective and there is a clear need for better vectors and better therapeutic genes. For cancer gene therapy, the ideal therapeutic gene should encode a protein that has the following properties:
1. Gives rise to a local bystander effect: i.e. the protein should be capable of killing the transduced tumour cell and its nontransduced neighbours.
2. Gives rise to a systemic bystander effect. Usually, this means that the treatment has the effect of enhancing the immune response against tumour antigens on distant tumour cells.
3. Selectivity. It is important that the treatment does not cause undue damage to normal (noncancerous) host tissues, especially the vital organs. Selectivity can be an intrinsic property of the protein encoded by the therapeutic gene and/or arise from its mode of action. Alternatively, or additionally, selectivity can be achieved by vector targeting to ensure that the therapeutic genes are not delivered to nontarget cells, or by the use of gene regulatory elements (promoters/enhancers/silencers/locus control sequences) that do not give rise to gene expression in nontarget cells.

The currently favoured classes of therapeutic gene are:
(1) Genes encoding proteins that enhance the immunogenicity of tumour cells. These include pro-inflammatory cytokines, T cell co-stimulators and foreign MHC proteins which produce a local bystander effect due to local inflammatory response. The local inflammatory response creates a cytokine-rich environment which favours the generation of a systemic bystander effect by recruitment and activation of tumour-specific T cells.
(2) Genes encoding enzymes that render tumour cells susceptible to a "pro-drug". For thymidine kinase gene transfer, there is some evidence for a local bystander effect due to transfer of ganciclovir triphosphate (the activated drug) through tight junctions to adjacent tumour cells. However, many rumours lack the requisite tight junctions and the observed local and systemic bystander effects are therefore presumed to arise because of a local inflammatory response to cells that are killed by the pro-drug with associated activation of tumour-reactive T cells.

Genes encoding engineered/targeted fusogenic viral membrane glycoproteins can satisfy all three criteria of local bystander effect, systemic bystander effect (by promoting a local inflammatory response which helps to amplify systemic immunity), and specificity and are better candidates than either of the previously mentioned groups of therapeutic genes but have not been previously used as therapeutic genes for cancer gene therapy. They have the capacity for generating a potent local bystander effect because they induce the fusion of gene-modified cells with surrounding nontransduced cells, resulting in the death of all the cells that have fused together. They can also be engineered to enhance their potential for triggering cell-cell fusion, and hence their therapeutic potency. Also, it is possible to engineer the specificity of the cell-cell fusion process by engineering the fusogenic proteins to ensure, for example, that circulating tumour cells that express the fusogenic proteins can fuse only with other tumour cells and do not therefore damage normal host tissues.

### Therapeutic genes encoding fusogenic membrane glycoproteins

Replicating viruses have been used extensively as oncolytic agents for experimental cancer therapy (Russell, 1994, Semin. Cancer Biol. 5, 437-443). Some of the viruses that have been used are known to encode fusogenic viral membrane glycoproteins. For example, a tissue culture suspension of mumps virus was used to treat 90 patients with terminal malignancies by local application to the tumour surface, by intratumoral, oral, rectal or intravenous inoculation, or by inhalation (Asada, 1974, Cancer, 34, 1907-1928). Toxicity was minimal and in 37 of the 90 patients the tumour disappeared or decreased to less than half of its initial size. Minor responses were observed in a further 42 patients. Tumour destruction was maximal several days after virus administration and was often followed by long-term suppression of tumour growth, perhaps due to stimulation of antitumour immunity.

Other viruses encoding fusogenic viral membrane glycoproteins that have been used for cancer therapy in human subjects or experimental mouse models include West Nile virus, herpes simplex virus, Russian Far East encephalitis, Newcastle disease virus, Venezuelan equine encephalomyelitis, rabies, vaccinia and varicella (Russell, 1994, Eur. J. Cancer, 30A, 1165-1171). The rationale for these studies has been that many viruses replicate and spread more rapidly in neoplastic tissues than in nontransformed tissues and might therefore be expected to cause more damage to the tumour than to the host.

It is possible that the fusogenic membrane glycoproteins of the viruses that have been used for cancer virotherapy may have contributed to the observed therapeutic effects by causing infected tumour cells to fuse with their uninfected neighbours. However, it has not previously been suggested that genes encoding fusogenic membrane glycoproteins should be removed from their natural context and inserted into a vector genome for use as a therapeutic formulation for cancer gene therapy.

### Summary of the Invention

In a first aspect the invention provides a pharmaceutical composition for use in gene therapy of a malignant disease comprising a recombinant nucleic acid vector in admixture with a pharmaceutically acceptable carrier, wherein the vector directs the expression on a eukaryotic cell surface of a fusogenic combination of separate paramyxoviral glycoproteins or a fusogenic membrane polypeptide which is other than a single paramyxoviral glycoprotein.

Conveniently, the vector directs the expression of a polypeptide which comprises at least a fusogenic portion of a viral fusogenic membrane glycoprotein (which may be abbreviated as FMG). In some embodiments, it is preferred that the syncytium-inducing polypeptide is capable of inducing syncytium formation at substantially neutral pH (i.e. pH6-8). Many suitable FMGs will be known to those skilled in the art and several are listed in the section of this specification entitled "Background of the Invention". A syncytium may be defined for present purposes as a cell-cell fusion which appears in a tissue biopsy or tissue culture sample as a multinucleate region of cytoplasm.

Typically the vector will be adapted so as to express the syncytium-inducing polypeptide on the surface of a human cell, such that, when properly expressed, the polypeptide may cause the cell to fuse with other human cells which do not express the syncytium-inducing polypeptide.

It is preferred that, where the polypeptide comprises a viral FMG, the FMG is expressed in substantial isolation from other viral components, except in the case where these are essential for fusogenic activity on target cells (e.g. the 'F' and 'H' glycoproteins of Paramyxoviridae both being required for syncytium-formation). The invention also therefore provides, in a further aspect, a pharmaceutical composition for use in gene therapy of a malignant disease comprising a combination of first and second recombinant nucleic acid vectors in admixture with a pharmaceutically acceptable carrier, wherein said first and second vectors, in combination, direct the expression on a eukaryotic cell surface of a fusogenic combination of separate paramyxoviral glycoproteins.

In addition, it will frequently be desirable to "engineer" the syncytium-inducing polypeptide to optimise its characteristics for therapeutic use, such that the vector directs the expression of a non-naturally occurring polypeptide. In particular embodiments the FMG will comprise at least a fusogeuic domain from a C-type retrovirus envelope protein, such as MLV or GaLV. A retroviral envelope protein with a deletion for all or most of the cytoplasmic domain may be preferred, as exhibiting hyperfusogenic activity for human cells.

Certain modifications can be introduced into viral membrane glycoproteins to enhance profoundly their ability to induce the formation of syncytia. For example, truncation of the cytoplasmic domains of a number of retroviral and herpesvirus glycoproteins has been shown to increase their fusion activity, sometimes with a simultaneous reduction in the efficiency with which they are incorporated into virions (Rein *et al*, J. Virol. 1994, 68, 1773-1781; Brody *et al*, J. Virol. 1994, 68, 4620-4627; Mulligan *et al*, J. Virol. 1992, 66, 3971-3975; Pique *et al*, J. Virol. 1993, 67, 557-561; Baghian *et al*, J. Virol. 1993, 67, 2396-2401; Gage *et al*, J. Virol. 1993, 67, 2191-2201). In addition, TM domain swapping experiments between MLV and HTLV-1 have shown that envelopes which are readily fusogenic in cell-to-cell assays and also efficiently incorporated into virions may not necessarily confer virus-to-cell fusogenicity (Denesvre *et al*, J. Virol. 1996. 70, 4380-4386).

By protein engineering, it is known to be possible to introduce novel binding specificities or protease-dependencies into fusogenic viral membrane glycoproteins and thereby to target their fusogenic activities to specific cell types that express the targeted receptors or to specific microenvironments that are rich in the appropriate activating proteases (See "Protease targets" below: also, Cosset & Russell, Gene Therapy, 1996, 3, 946-956).

### Protease targets

There appears to be a large number of membrane proteases which are preferentially expressed on the surfaces of tumour cells. They have been implicated in a variety of processes that contribute to disease progression and treatment resistance such as invasion, metastasis, complement resistance.

Complement resistance: the human melanoma cell line SK-MEL-170 is resistant to complement-mediated lysis. The molecular basis for this complement resistance has been defined as a membrane protease p65 which rapidly and specifically cleaves C3b deposited on the SK-MEL-170 cell surface (Ollert *et al*, Cancer Res. 1993, 53, 592-599).

Prostate-specific antigen: ejaculated semen is immediately turned into a viscous gel which liquifies within 20 minutes. PSA is a prostatic kallikrein-like serine protease which participates in this liquefaction process by cleaving semenogelin, the predominant protein in the coagulated part of the ejaculate (Lilja *et al*, J. Clin. Invest. 1987, 80, 281-285). PSA is produced exclusively by prostatic epithelial cells and is a useful marker for prostatic cancer. PSA has also been shown to cleave IGFBP-3, greatly reducing its affinity for insulin-like growth factor (IGF-1) (Cohen *et al*, J. Endocrinol. 1994, 142, 407-415). PSA circulating in plasma is inactive because it is bound to serpins but it has been postulated that local release of PSA in metastatic foci of prostatic cancer might lead to the release of IGF1 by cleaving IGFBP binding protein 3 thereby enhancing tumour growth (Cohen *et al* J. Endocrinol. 1994 Vol. 142 p 407-415).

Procoagulant proteases: deposition of fibrin on cancer cells may protect them from the immune system and participation of coagulation enzymes in metastasis has been suggested (Dvorak, Hum. Pathol. 1987, 18, 275-284). Membrane-associated procoagulants which may be of significance in this respect include tissue factor (Edwards *et al,* Thromb. Haemostasis. 1993, 6, 205-213), an enzyme that directly activates factor X (Gordon & Cross, J. Clin. Invest. 1981, 67, 1665-1671) and a protease that directly converts prothrombin to active thrombin (Sekiya *et al*, J. Biol. Chem. 1994, 269, 32441-32445).

Plasminogen activation system: plasmin is a broad spectrum trypsin-like protease that degrades fibrin and ECM proteins including laminin, thrombospondin and collagens and that activates other latent matrix-degrading proteases such as collagenases. The expression of protease activity by tumour cells is proposed to facilitate their penetration of basement membranes, capillary walls, and interstitial connective tissues, allowing them to spread to other sites and establish metastases (Dano *et al*, Adv. Cancer Res. 1985, 44, 139-266). Plasminogen is an abundant plasma protein (Mr = 90,000) normally present at a concentration of about 2µM. Most cell types analysed, except erythrocytes, have a high density of low affinity (0.1-2.0 µM) plasminogen binding sites which recognise the lysine binding sites associated with the kringle domains of plasminogen (Redlitz & Plow, Clin. Haem. 1995, 8. 313-327). Cell-bound plasminogen is activated by a single peptide bond cleavage to form plasmin which is composed of a disulphide-linked heavy chain (Mr = 60,000, containing five kringle motifs) and light chain (Mr = 24,000 containing the seine proteinase catalytic triad). Activation of plasminogen to plasmin is mediated primarily by cell-bound u-PA or t-PA (see below). Cell bound plasmin is more active than soluble plasmin and is resistant to inactivation by the alpha-2-antiplasmin present in serum, but is rapidly inactivated after dissociation from the cell (Stephens *et al*, J. Cell Biol. 1989. 108. 1987-1995).

Urokinase plasminogen activator (u-PA) is involved in cell-mediated proteolysis during wound healing, macrophage invasion, embryo implantation, signal transduction. invasion and metastasis. Pro-uPA is usually released by cells as a single-chain of 55kDa (scuPA), and binds to its GPI-anchored cellular receptor (uPAR - Kd 0.05 - 3.0nM) where it is efficiently converted to its active (two-chain) form by plasmin or other proteases.

Thrombin inactivates the active form of u-PA (Ichinose *et al*, J. Biol. Chem. 1986, 261, 3486-3489). The activity of cell-bound u-PA is regulated by three inhibitors, PAI-1, PAI-2 and protease nexin (PN) which can bind to the cell-bound enzyme resulting in its endocytic sequestration from the cell surface (Conese and Blasi, Clin. Haematol. 1995, 8, 365-389).

In cancer invasion there appears to be a complex interplay between the various components of the plasminogen-plasminogen activator system. uPAR clustering on the cell surface serves to focus the process of plasmin-mediated pericellular proteolysis at the invading front of the tumour. pro-u-PA, uPAR, PAI-1 and PAI-2 can be produced in varying amounts by the cancer cells, or by nontransformed stromal cells at the site of tumour invasion and their production by these different cell types can be regulated by a variety of stimuli (Laug *et al*, Int. J. Cancer, 1992, 52, 298-304; Ciambrone & Mckeown-Longo, J. Biol. Chem. 1992, 267, 13617-13622; Kessler & Markus, Semin. Thromb. Haemostasis, 1991, 17, 217-224; Lund *et al*, EMBO J., 1991, 10, 3399-3407). Thus, various different cell types can contribute to the assembly on the tumour cells of all the components of the proteolytic machinery that is required for matrix destruction.

Trypsin-like proteases: tumour-associated trypsin inhibitor (TATI) is a 6-kDa protease inhibitor whose levels are elevated in patients with advanced cancer (Stenman *et al*, Int. J. Cancer, 1982, 30, 53-57). In search of the target protease for the TATI, two trypsin-like proteases have been purified from the cyst fluid of mucinous ovarian tumours (Koivunen *et al*, J. Biol. Chem. 1989, 264, 14095-14099). Their substrate specificities were found to be very similar to those of pancreatic trypsins 1 and 2 and they were found to be efficient activators of pro-urokinase but could not activate plasminogen directly.

Cathepsin D: this is a pepstatin-sensitive lysosomal aspartyl protease which is secreted in large amounts by breast cancer cells and by a variety of other cancer cell types. Purified cathepsin D and conditioned medium from cathepsin D-secreting cells have been shown to degrade extracellular matrix at pH 4.5, but not at neutral pH (Briozzo *et al*, Cancer Res. 1988, 48, 3688-3692). It has therefore been proposed that the enzyme may be an important facilitator of tumour invasion when it is released into an acidic (pH < 5.5) microenvironment. One factor distinguishing it from other protease classes is that it can act at a distance from the cancer cell after it has been secreted.

Cathepsin B. L: leupeptin-sensitive lysosomal cysteinyl proteases which act at acidic pH.

Thus possible characteristics of viral FMGs which may be susceptible to improvement by protein engineering include:-
(1) pH at which fusion is mediated (as explained elsewhere, many viral FMGs mediate fusion only at acid pH, whereas fusion at neutral pH may frequently be preferred);
(2) activation of the fusion function upon exposure to certain proteases (this can lead to localised activation at the surface of, or in the vicinity of, tumour cells, many of which secrete or express tumour-associated proteases, as explained in the section of the specification entitled "Protease targets" - accordingly the FMG can be targeted to tumour cells);
(3) modification of natural FMGs (e.g. amino acid substitutions, truncations, or production of chimeric FMGs) - chimeric FMGs could comprise novel binding specificities to target the FMGs to particular cell surface markers, or combine other desirable characteristics from different proteins.

In summary, there are many known viral membrane glycoproteins or combinations of viral membrane glycoproteins which, when expressed on the surface of a mammalian cell, are capable of causing that cell to fuse with neighbouring cells that do not express the viral membrane glycoproteins, to form a nonviable syncytium. The proteins that mediate this process can be engineered for enhanced fusogenic activity (e.g. by deletion of part or all of the cytoplasmic domains thereof), altered receptor specificity (e.g. by fusion to polypeptides with other binding specificities), or novel protease-dependency.

The composition of the invention may be used in a method (outside the scope of the present claims) of treating a malignant disease in a human patient, comprising administering to the patient a recombinant nucleic acid directing the expression of a syncytium inducing polypeptide in a human cell, such that cells ("index" cells) of the patient which take up the recombinant nucleic acid will fuse with cancerous cells ("target" cells) causing the malignant disease.

In particular the nucleic acid may be introduced *in vitro* into suitable human index cells (by any one of various known standard techniques, such as transfection, transduction or transformation), and the index cells are then introduced into the patient, where they can exert a fusogenic effect on target cells.

The FMGs of use in the invention can be used in different ways for cancer gene therapy. The primary target cells in which the FMGs are expressed (index cells) can be stationary cells (e.g. the neoplastic cells or stromal elements in a solid tumour) or migratory cells (e.g. T lymphocytes, B lymphocytes and other haemopoietic cells or migratory neoplastic cells in haematological malignancies). The secondary target cells (with which the FMG-expressing target cells will fuse) may be likewise be stationary or migratory. The target cells can be transduced *ex vivo* or *in vivo* by the FMG-encoding vectors. Any vector system, whether viral or nonviral can be used to deliver the FMG genes to the target cells. Targeting elements may be included in the vector formulation to enhance the accuracy of gene delivery to the target cells and tissue/tumour-selective regulatory elements can be included in the vector genome to ensure that the expression of the FMG genes is restricted to the chosen target cells.

Genes encoding FMGs could therefore be used in various ways for therapeutic benefit. The aim in all cases is to destroy unwanted target cells by causing them to fuse with FMG-expressing index cells. The initial targets for gene transfer are therefore the index cells, but the ultimate targets of the therapeutic strategy are the cells with which they fuse. Many different therapeutic strategies can be envisaged.

For example, where the aim of the protocol is to destroy neoplastic cells in the patient, the index cells need not be neoplastic. Migratory T lymphocytes expressing tumour-selective FMGs might form syncytia exclusively with neoplastic cells. Local expression of tumour-selective (or, less optimally, nonselective) FMGs in the stromal, vascular endothelial or neoplastic cells in solid tumours might lead to recruitment of neighbouring neoplastic cells into syncytia.

For leukaemias and other haematogenous malignancies, expression of leukaemia-selective FMGs in vascular endothelium or stromal bone marrow cells might lead to recruitment of circulating leukaemic cells into stationary syncytia. Alternatively, expression of leukaemia-selective FMGs in circulating T cells or in the leukaemic cells themselves might allow these cells to nucleate the formation of leukaemic cell syncytia in heavily infiltrated tissues, or lead to recruitment of leukaemic cells into recirculating syncytia.

In a further aspect the invention provides for use of a recombinant nucleic acid vector in the preparation of a medicament to treat a malignant disease in a human patient, the vector directing the expression on a eukaryotic cell surface of a fusogenic combination of separate paramyxoviral glycoproteins or a fusogenic membrane polypeptide which is other than a single paramyxoviral glycoprotein.

Compositions in accordance with the invention typically comprise a host cell comprising a recombinant nucleic acid vector as described above. The cell will typically be a eukaryotic cell (especially a human cell) and desirably will express on its surface a syncytium-inducing polypeptide. The pharmaceutical compositions of the invention typically comprise a recombinant nucleic acid vector in admixture with a pharmaceutically acceptable carrier or a eukaryotic cell containing a recombinant nucleic acid vector in admixture with a pharmaceutically acceptable carrier.

A vector of use in the invention may be administered directly to a patient, or may be administered utilizing an *ex vivo* approach, whereby cells are removed from a patient or donor, transduced with the vector and re-implanted into the patient. The vector, or host cells containing the vector, may be administered prophylactically, or to patients having a malignant disease or other condition appropriate for treatment. Administration may comprise the use of a delivery vehicle (e.g. a liposome) or may involve iontophoresis. electroporatiun or other pharmacologically approved delivery method. Routes of administration may include intramuscular, intravenous, aerosol, oral, topical, systemic, ocular or intraperitoneal, amongst others.

The invention will now he further described by way of illustrative example and with reference to the accompanying drawings in which:
Figures 1 - 3 are schematic representations of recombinant nucleic acid vectors: in figure 2 CMV is the CMV promoter; in Figures 1 and 3 LTR is the long terminal repeat; in Figure 3 phleo^{r} is the phleomycin resistance gene: in Figures 2 and 3 the IEGR linker sequence is the protease cleavage signal for FXa protease and * denotes stop codons;
Figure 4 is an immunoblot of cell lysates prepared from TELCeB6 transfectants, pFBH, pFBH EGF^{R-}, pFBH XEGF^{R-}, pFBH IGF, pFBH XIGF and the control, untransfected TELCeB6, probed with an anti-MV H antiserum;
Figure 5 shows a magnified view showing large C170 syncytia in a cell-cell fusion assay after X-gal staining: chimaeric MV H proteins show syncytia formation, although at a lower level to that of the unmodified H protein;
Figure 6 shows the DNA and amino acid sequence of a truncated hyperfusogenic GaLV envelope protein; and
Figure 7 is a schematic representation of further recombinant nucleic acid vectors: in figure 7, the striped box is the FXa cleavage signal, the lightly shaded box is the mature (residues 43-653 only) GaLV envelope, and the heavily shaded box is residues 633-674 of the moloney MLV envelope, poly A is a polyadenylation signal, L is a leader sequence.

### Examples

Demonstration of the therapeutic use of genes encoding (targeted) fusogenic membrane glycoproteins for gene therapy of cancer.

When expressed concurrently in the same cell, measles virus F and H glycoproteins can mediate cell-cell fusion with neighbouring cells, provided the neighbouring cells express the measles virus receptor (CD46). Human cells express the CD46 measles virus receptor, whereas murine cells do not. In the experiments described below, a retroviral vector capable of transferring the measles virus F and H genes is used to demonstrate the therapeutic potential of gene therapy vectors encoding targeted or nontargeted fusogenic viral membrane glycoproteins for cancer therapy. The vectors can be used for direct gene transfer to tumour cells or for transduction of nontumour cells which are then employed for their selective antitumour effect.

### Example 1 Construction of retroviral vector plasmid coding for Measles virus F and H glycoproteins.

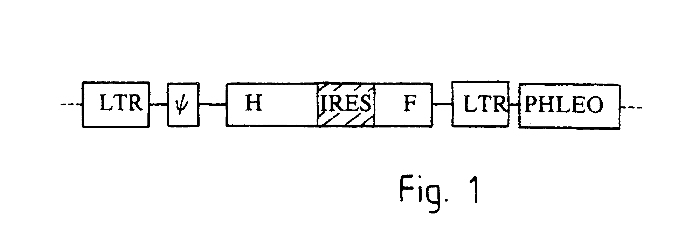

The plasmid shown schematically in Figure 1 (not to scale) is constructed using standard cloning methods. In relation to figure 1, LTR = Moloney murine leukaemia virus long terminal repeat; ψ = Moloney murine leukaemia virus packaging signal; IRES = poliiovirus internal ribosome entry site; H = measles virus H glycoprotein coding sequence; F = measles virus F glycoprotein coding sequence; PHLEO = phleomycin resistance marker; the dotted line represents the vector backbone (either pUC or pBR322-based). In brief, the coding sequence of the measles virus H gene is cloned from pCGH5 (Cathomen *et al*, 1995, Virology, 214, 628-632), into the *Not*I site of the retroviral vector plasmid pGCP (which contains the poliovirus internal ribosome entry site flanked by *Not*I and *Cla*I cloning sites). The measles virus F gene is then cloned from pCGF (Cathomen *et al*, 1995, Virology, 214, 628-632) into the *Cla*I site of the same vector, 5' of the internal ribosome entry site to produce the vector named pHF. A phleomycin selectable marker gene is then introduced into the vector 5' of the 5' LTR.

### 1.2 Preparation of retroviral vector stocks.

The plasmid pHF is transfected into amphotropic retroviral packaging cell lines which were derived from murine fibroblasts. Suitable packaging cell lines are widely available and include the NIH 3T3-derived cell lines PA317 and GP+env AM12. Stably transfected packaging cells are selected in phleomycin 50µg/ml and used as a source of HF retroviral vectors capable of efficiently transferring the measles virus F and H genes to human and murine target cells.

### 1.3 Transduction of transplantable human tumour cell lines leading to formation of multinucleated syncytia through the induction of cell-cell fusion.

The HF retroviral vector stocks are used to transduce a panel of human tumour cell lines which are subsequently observed for the formation of multinucleated syncytia, expected to be maximal 24 to 72 hours after retroviral transduction of the cells. The tumour cell lines are grown to near-confluency before transduction. Examples of tumour cell lines that can be used for this assay are A431 (epidermoid carcinoma), HT1080 (fibrosarcoma), EJ (bladder carcinoma), C175 (colon carcinoma), MCF7 (breast carcinoma), HeLa (cervical carcinoma), K422 (follicular lymphoma), U266 (myeloma). Most, if not all, of the human tumour cell lines tested undergo extensive cell-cell fusion shortly after transduction with the HF retroviral vector.

### 1.4 Inoculation of nude mice with transplantable human tumour cell lines and subsequent in vivo transfer of H and F genes to the rumour deposits: Demonstration that fusogenic membrane glycoproteins mediate tumour destruction in the absence of a functional immune system.

Mice are challenged by subcutaneous inoculation into the flank with 10⁷ human tumour cells. Suitable cell lines for use in these experiments are listed above in Section 3. Between one and fourteen days after subcutaneous inoculation with tumour cells, the growing tumour xenografts are inoculated with concentrated HF retroviral vector stocks or by control vector stocks encoding either measles F or measles H glycoproteins. Tumour growth is slowed or completely inhibited by HF retroviral vector inoculation but not by inoculation of control (H or F alone) vectors.

### 1.5 Transduction of murine fibroblasts; lack of cell-cell fusion and absence of multinucleated syncytia.

The HF retroviral vector stocks are used to transduce murine NIH3T3 fibroblasts which are subsequently observed for the formation of multinucleated syncytia. No cell-cell fusion occurs and no multinucleated syncytia are observed.

### 1.6 Mixing of HF-transduced murine fibroblasts with nontransduced human tumour cells leading to the formation of multinucleated syncytia through the inducation of cell-cell fusion between HF-transduced murine fibroblasts and nontransduced human tumour cells.

The HF retroviral vector stocks are used to transduce murine NIH3T3 fibroblasts which are subsequently mixed, at various ratios from 1:1 to 1:10,000, with nontransduced human tumour cell lines. The mixed cell populations are then plated at high density and observed for the formation of multinucleated syncytia. Cell-cell fusion occurs between HF-transduced NIH3T3 fibroblasts and nontransduced human tumour cells leading to the formation of multiple hybrid syncytia, each one nucleating on a transduced NIH3T3 cell. Syncytia are not observed in control cultures in which nontransduced NIH3T3 cells are mixed with nontransduced human tumour cells.

### 1.7 Inoculation of nude mice with mixtures of HF-transduced murine fibroblasts and nontransduced human tumour cells: Demonstration that fusogenic membrane glycoprotein-expressing cells mediate tumour destruction by recruitment into syncytia of nontransduced human tumour cells.

The HF retroviral vector stocks are used to transduce murine NIH3T3 fibroblasts which are subsequently mixed, at varying ratios from 1:1 to 1:10,000, with nontransduced human tumour cell lines. Mixed cell populations containing 10⁷ tumour cells admixed with from 10³ to 10⁷ HF-transduced NIH3T3 cells are then inoculated subcutaneously into the flanks of nude (BALBC nu/nu) mice and the mice are monitored for the growth of subcutaneous tumours whose diameters are recorded daily. Control mice are challenged with 10⁷ nontransduced human tumour cells. Tumour growth is slowed or completely inhibited by admixed HF-transduced NIH3T3 fibroblasts which express the measles virus F and H glycoproteins, but not by admixed nontransduced NIH3T3 fibroblasts.

### Example 2 Display of EGF and IGF on Measles H Glycoprotein

### MATERIALS AND METHODS

### Plasmid Construction

Unmodified Measles Virus (MV) F and MV H protein were encoded by the expression plasmids pCG-F and pCG-H, respectively (Catomen et al, Virology 214 p628, 1995). To make the chimaeric MV H expression constructs, first the *Sfi*I site in pCG-H was deleted, so that we could introduce our displayed ligands as *Sfi*I/*Not*I fragments. This was done by digesting pCG-H with *Sfi*I, endfilling the cohesive ends using Klenow fragment of *E. coli* DNA polymerase and dNTPs, then re-ligating the purified product. This construct was tested to check that it was still functional in cell fusion assays (see later). We could now make constructs which would enable us to insert ligands as *Sfi*I/*Not*I fragments. To make the construct pCG-H *Sfi*I/*Not*I, which introduces the *Sfi*I/*Not*I cloning site at the C-terminus of the MV H sequence, oligonucleotides HXmabak (5'-CCG GGA AGA TGG AAC CAA TGC GGC CCA GCC GGC CTC AGG TTC AGC GGC CGC ATA GTA GA-3', Seq ID No. 1) and HSpefor (5'-CTA GTC TAC TAT GCG GCC GCT GAA CCT GAG GCC GGC TGG GCC GCA TTG GTT CCA TCT TC-3', Seq ID No. 2) were made. When annealed together these two oligonucleotides form a DNA fragment with *Xma*I and *Spe*I cohesive ends. This fragment was ligated to the *Xma*I/*Spe*I digested pCG-H(Sfi-) backbone. The correct sequence of the construct was verified by DNA sequencing.

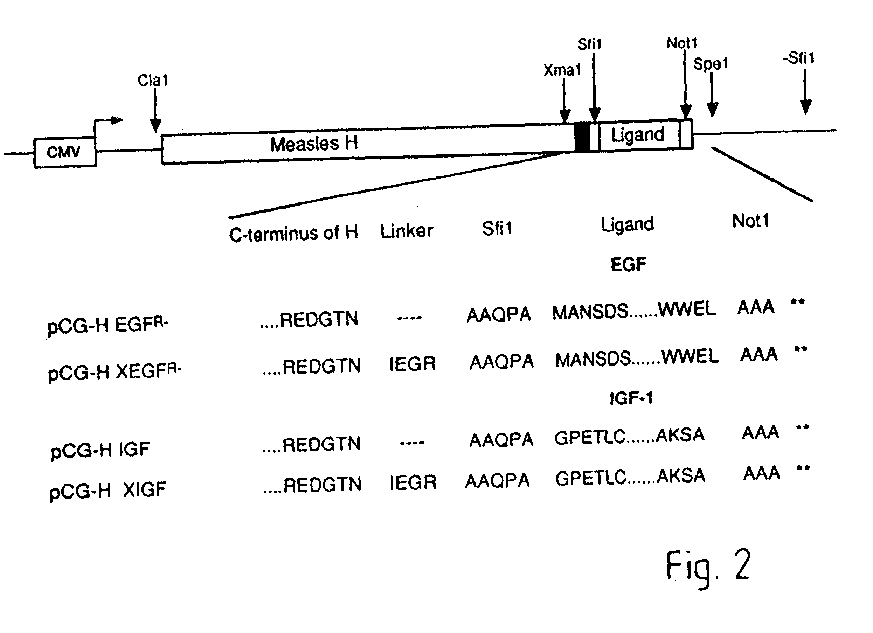

To make the construct pCG-H FX*Sfi*I/*Not*I, where there is a FXa protease cleavage signal before the *Sfi*I/*Not*I cloning sites at the C-terminus of the MV H sequence, oligonucleotides HXmaFXbak (5'-CCG GGA AGA TGG AAC CAA TAT CGA GGG AAG GGC GGC CCA GCC GGC CTC AGG TTC AGC-3', Seq ID No. 3) and HNotFXfor (5'-GGC CGC TGA ACC TGA GGC CGG CTG GGC CGC CCT TCC CTC GAT ATT GGT TCC ATC TTC-3', Seq ID No. 4) were made. When annealed together these two oligonucleotides form a DNA fragment with *Xma*I and *Not*I cohesive ends. This fragment was ligated to the *Xma*I/*Not*I digested pCG-H *Sfi*I/*Not*I backbone. The correct sequence of the constructs was verified by DNA sequencing. Constructs pCG-H EGF^{R-}, pCG-H XEGF^{R-}, pCG-H IGF and pCG-H XIGF were made by transferring the *Sfi*I/*Not*I EGF and IGF fragments from pEGF^{R-}GS1A1 (Peng, PhD Thesis) and pIGFA1 (IA) (WO97/03357, Russell et al.) respectively into *Sfi*I/*Not*I digested pCG-H *Sfi*I/*Not*I and pCG-H FX*Sfi*I/*Not*I. Figure 2 shows a diagrammatic representation of the four constructs.

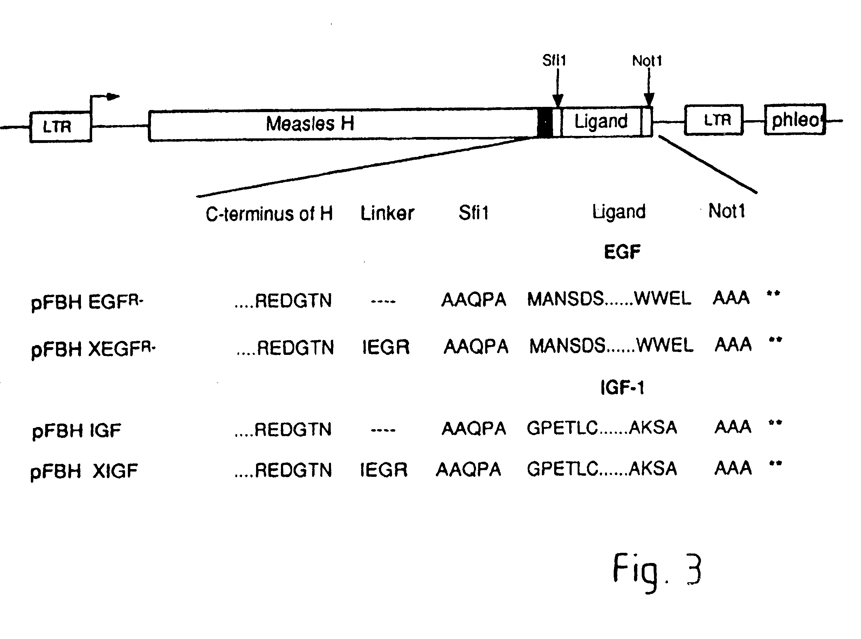

To enable us stably to express the chimaeric H proteins in mammalian cells, we need to have a selectable marker in the expression construct. This was achieved by transferring the whole MV H gene with the *Sfi*I/*Not*I cloning site at its C-terminus into the envelope expression construct, EMo1 (Cosset et al, J. Virol. 69 p6314, 1995). So, to make pFBH *Sfi*I/*Not*, pCG-H *Sfi*I/*Not* was cut with *Cla*I and *SpeI* to release the H gene with the *Sfi*I/*Not*I cloning site and EMo1 was cut with *Xba*I and *Cla*I to remove EGF and the Mo envelope sequence giving us the backbone. The cohesive ends of both fragments were endfilled using Klenow fragment of *E*. *coli* DNA polymerase and dNTPs. The backbone was phosphatased and the purified fragments were ligated together. The construct was checked by diagnostic digests for the correct orientation. To make the construct pFBH FX*Sfi*I/*Not*, pCG-H FX*Sfi*I/*Not* was cut with *Nsi*I and *Not*I to release part of the H sequence with a FXa protease cleavage signal and the *Sfi*I/*Not*I cloning site at its C-terminus. pFBH *Sfi*I/*Not* was also cut with *Nsi*I and *Not*I to give us the backbone, and the two fragments were ligated together. The construct was checked by sequencing for correctness. Constructs pFBH EGF^{R-}, pFBH XEGF^{R-}, pFBH IGF and pFBH XIGF were made by transferring the *Sfi*I/*Not*I EGF and IGF fragments from pEGF^{R-}GS1A1 and pIGFA1 respectively into *Sfi*I/*Not*I digested pFBH *Sfi*I/*Not* and pFBH FX*Sfi*I/*Not*I. Figure 3 shows a diagrammatic representation of the four constructs. To make the construct pFBH, where there is no C-terminal extension, pCG-H was cut with *Cla*I and *Spe*I to release the H gene and EMo1 was cut with *Xba*I and *Cla*I to remove EGF and the Mo envelope sequence giving us the backbone. The cohesive ends of both fragments were endfilled using Klenow fragment of *E*. *coli* DNA polymerase and dNTPs. The backbone was phosphatased and the purified fragments were ligated together. The construct was checked by diagnostic digests for the correct orientation.

### Cell lines

C170 cells, a human colon cancer cell line (Durrant et al, Br. J. Cancer 53 p37, 1986), and Human A431 cells (ATCC CRL1555) were grown in DMEM supplemented with 10% fetal calf serum. To enable easy detection of cell-cell fusion the C170 and A431 cells were infected with A viral supernatant, harvested from TELCeB6 producer cells (Cosset et al, J. Virol. 69 p6314, 1995), which transfers a gene coding for β-galactosidase tagged with a nuclear localisation signal. Single colonies of cells were grown up and clones that stained blue were picked. These blue staining C170 and A431 cells were used in cell fusion assays. The different MV H expression constructs pFBH, pFBH EGF^{R-}, pFBH XEGF^{R-}, pFBH IGF and pFBH XIGF (5mg DNA) were transfected into TELCeB6 cells (Cosset et al, J. Virol. 69 p7430, 1995) using 30ml Superfect (Qiagen). Stable phleomycin (50mg/ml) resistant colonies were expanded and pooled. Cells were grown in DMEM supplemented with 10% fetal calf serum.

### Immunoblots

To obtain cell lysates, TELCeB6 cells stably transfected with the MV H constructs were lysed in a 20mM Tris-HCl buffer (pH 7.5) containing 1% Triton X-100, 0.05% SDS, 5mg/ml sodium deoxycholate, 150mM NaCl and 1mM phenylmethylsulfonylfluoride. Lysates were incubated for 10 mins at 4°C and then centrifuged for 10 mins at 10,000 x g to pellet the unwanted nuclei. Aliquots of the cell lysates (50µl) were then separated on a 10% polyacrylamide gel under reducing conditions followed by transfer of the proteins onto nitrocellulose paper (NC) (Amersham). The NC was blocked with 5 % skimmed milk powder (Marvel) in PBS-0.1%Tween 20 (PBST) for 30 mins at room temperature. The MV H proteins were detected by incubating the NC for 3 hours with a MV H specific rabbit serum (1 in 3000) which was raised against a peptide derived from the amino terminus of the H protein (kind gift from Roberto Cattaneo, University of Zurich). After extensive washing with PBST the NC was incubated with horseradish peroxidase-conjugated swine anti-rabbit antibodies (1 in 3000) (DAKO, Denmark) for 1 hour at room temperature. Proteins were visualised using the enhanced chemiluminescence kit (Amersham Life Science, UK).

### Cell-cell fusion assays

Blue staining C170 and A431 cells were seeded at 5 x 10⁵ cells/well in six-well plates and incubated at 37°C overnight. MV H expression constructs, pCG-H, pCG-H EGF^{R-}, pCG-H XEGF^{R-}, pCG-H IGF and pCG-H XIGF, were co-transfected into the C170 and A431 cells along with the MV F expression construct, pCG-F. Transfections were carried out using 2.5mg of the relevant plasmids and 15ml Superfect. After transfection the cells were incubated with regular medium for 48-72 hrs, until syncytia could be clearly seen. X-Gal staining for detection of β-galactosidase activity was performed as previously described (Takeuchi *et al*., 1994). Fusion efficiency was scored (- no syncytia, + definite syncytia, ++ abundant syncytia).

### RESULTS

### Construction of chimaeric MV H expression constructs

A series of expression constructs were made which code for chimaeric MV H proteins in which the ligands EGF and IGF are fused at the C-terminus of the H protein with or without a Factor Xa-cleavable linker (Figures 2 and 3). Figure 2 shows constructs which are driven by the CMV promoter, but these constructs contain no selectable marker for selection in mammalian cells. Expression of the constructs in Figure 3 is driven by a retroviral LTR and these constructs contain the selectable marker, phleomycin, for selection in mammalian cells.

### Expression of the chimaeric MV H proteins

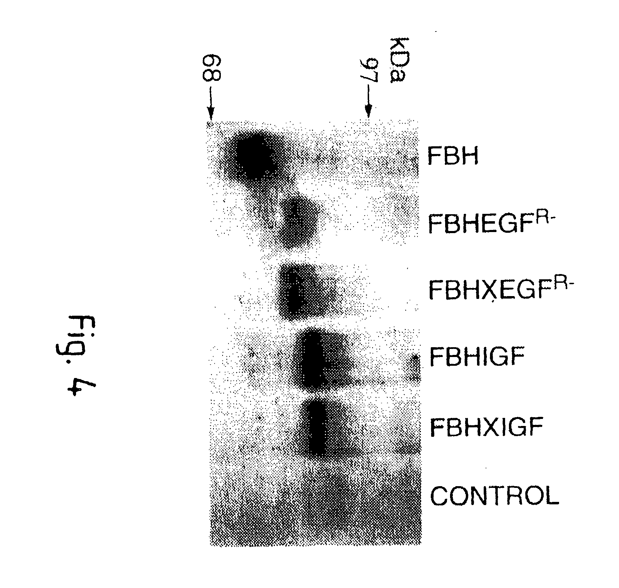

The different MV H expression constructs, pFBH, pFBH EGF^{R-}, pFBH XEGF^{R-}, pFBH IGF and pFBH XIGF were stably transfected into TELCeB6 cells. Immunoblots were performed on cell lysates prepared from these stable TELCeB6 transfectants. Figure 4 shows that all chimaeric MV H proteins are expressed to a comparable level to that of the wild type MV H protein. Moreover, the blot shows that the displayed domains are not spontaneously cleaved from the chimaeric MV H glycoproteins.

### Cell-cell fusion assays

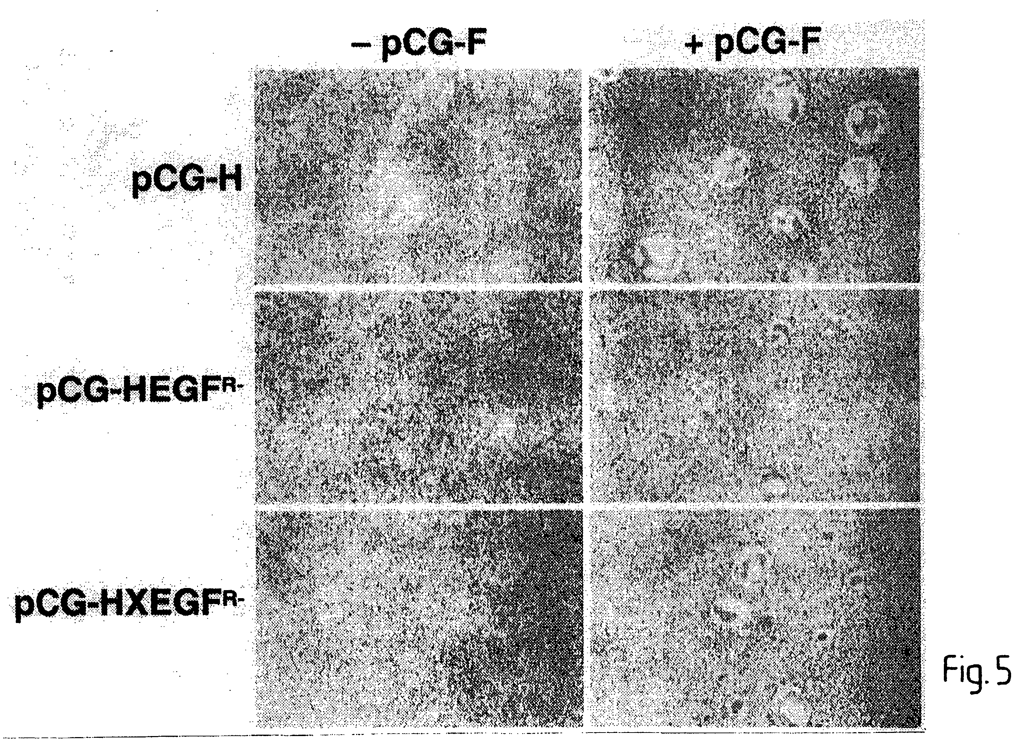

MV H expression constructs, pCG-H, pCG-H EGF^{R-}, pCG-H XEGF^{R-}, pCG-H IGF and pCG-H XIGF, were co-transfected into the β-galactosidase expressing C170 and A431 cells along with the MV F expression construct, pCG-F. The cells were stained with X-gal substrate 72 hrs after transfection to allow ease of cell-cell fusion detection. Results of the assays are shown in Tables 1 and 2 and in Figure 5. The chimaeric MV H proteins were potent inducers of cell-cell fusion in C170 cells although their potency was slightly reduced compared to the unmodified H protein (Table 1, Figure 5). Cell-cell fusion in A431 was abolished for the chimaeric H proteins compared to the unmodified MV H protein which was a potent inducer of cell-cell fusion (Table 2).

The results show that:
1) Foreign polypeptides can be displayed as fusions to the extreme C-terminus of the MV H protein.
2) The chimaeric H glycoproteins are efficiently expressed and are functional in cell-cell fusion assays.
3) The displayed ligand can target the specificity of cell-cell fusion.

**Table 1**

| This table shows the results of cell-cell fusion on β-galactosidase expressing C170 cells. Chimaeric MV H proteinsare potent inducers of cell-cell fusion when co-expressed with unmodified F glycoproteins. - =no syncytia, + = definite syncytia, ++ = abundant syncytia. | | |
|---|---|---|
| | **-pCG-F** | **+ pCG-F** |
| pCG-H | - | ++ |
| pCG-H EGF | - | ++ |
| pCG-H XEGF | - | ++ |

**Table 2**

| This table shows the results of cell-cell fusion assay on β-galactosidase expressing A431 cells. The unmodified MV H protein is a potent inducer of cell-cell fusion when co-expressed with with unmodified F glycoproteins. However, chimaeric MV H proteins show no syncytia formation. - =no syncytia, + = definite syncytia, ++ = abundant syncytia. | | |
|---|---|---|
| | **-pCG-F** | **+ pCG-F** |
| pCG-H | - | ++ |
| pCG-H EGF | - | - |
| pCG-H XEGF | - | - |

### Example 3 Demonstration that GALV envelope with truncated cytoplasmic tail is hyperfusogenic on human tumour cell lines

### MATERIALS AND METHODS

### Plasmids Used

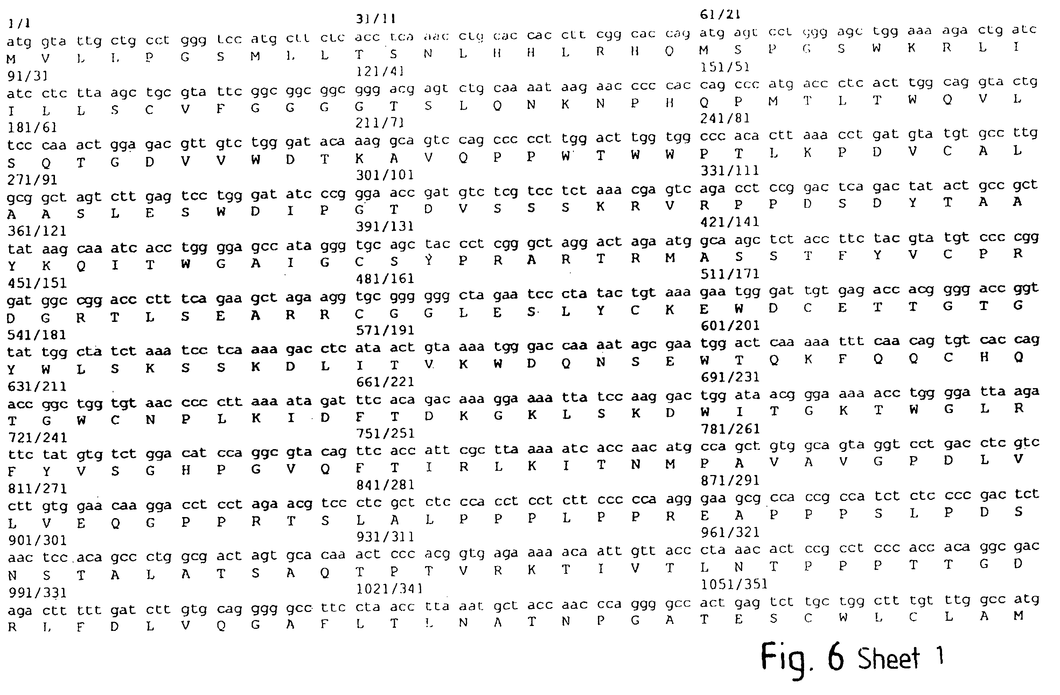

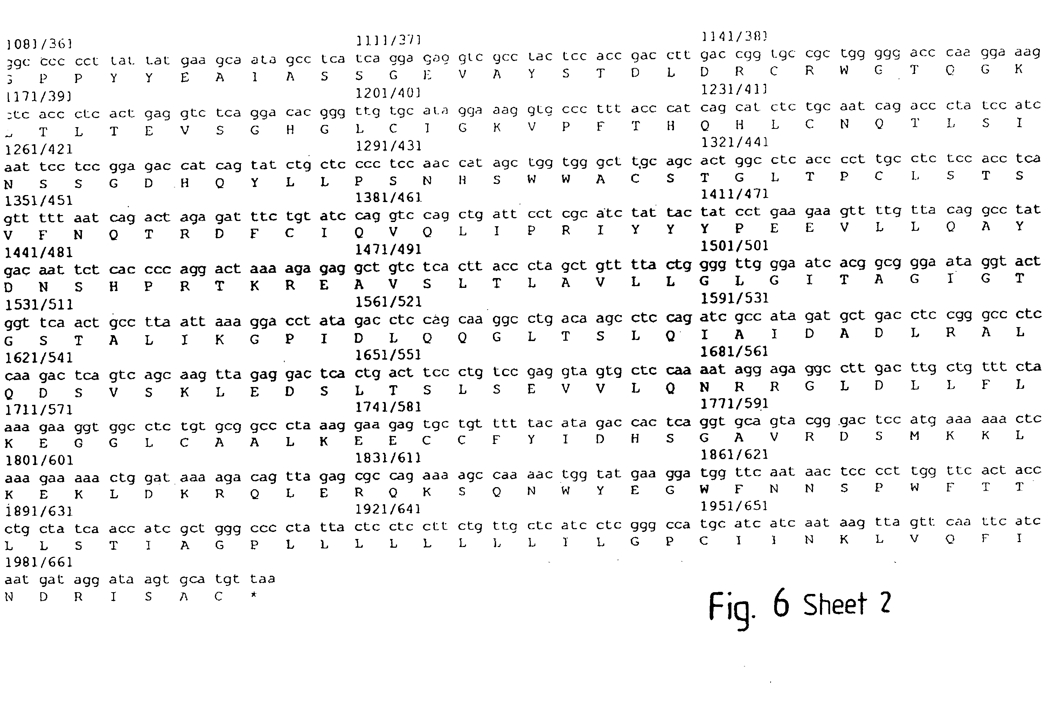

The expression constructs of Measles Virus (MV) F and MV H protein were encoded by the expression plasmids pCG-F and pCG-H, respectively (Catomen et al, Virology 214 p628, 1995). FBdelPGASAF encodes the wildtype GALV envelope and FBdelPGASAF-fus encodes a C-terminally truncated GALV envelope lacking the cytoplasmic tail (see attached sequence, Figure 6).

### Cell lines

Human C170 (Durrant et al, Br. J. Cancer 53 p37, 1986), Human A431 cells (ATCC CRL1555), Human TE671 (ATCC CRL8805), Human Hela (ATCC CCL2), and the murine cell line NIH3T3 were grown in DMEM supplemented with 10% fetal calf serum. All of these cell lines, except NIH3T3 have receptors for the GALV envelope and for the MV H glycoprotein.

### Cell-cell fusion assays

Cells were seeded at 5 x 10⁵ cells/well in six-well plates and incubated at 37°C overnight.

The fusogenic and non-fusogenic plasmids, FBdelPGASAF and FBdeIPGASAF-fus, were transfected and the MV H and F expression constructs, pCG-H and pCG-F, were co-transfected into the panel of cell lines. Transfections were carried out using 2.5mg of the relevant plasmids and 15ml Superfect (Qiagen). After transfection the cells were incubated with regular medium for 48-72 hrs, until syncytia could be clearly seen, when fusion efficiency was scored (- no syncytia, + definite syncytia, ++ abundant syncytia).

### RESULTS

### Cell-cell fusion assays

The fusogenic and non-fusogenic plasmids and the MV H and F expression constructs were transfected into the panel of cell lines. The cells were left for 72 hours before cell-cell fusion was scored. Results of the assays are shown in Table 3. The fusogenic GALV construct shows the same pattern of fusion ability as the MV F and H proteins show.

**Table 3**

| This table shows the results of cell-cell fusion assays on a panel of cell lines. - = no syncytia, + = definite syncytia, + + = abundant syncytia. | | | |
|---|---|---|---|
| | **FBdelPGASAF** | **FBdeIPGASAF-fus** | **CG-F/CG-H** |
| C170 | - | ++ | ++ |
| A431 | - | ++ | ++ |
| TE671 | - | ++ | ++ |
| HeLa | - | ++ | ++ |
| NIH3T3 | - | - | - |

### Example 4 Display of EGF on GALV Envelope

### MATERIALS AND METHODS

### Construction of envelope expression plasmids

Envelope expression plasmid GALVMoT was constructed by PCR amplification of the cDNA encoding GaLV env from the plasmid pMOVGaLVSEATO env (Wilson et al., J. Virol. 63, 2374-2378, 1989) using primers GalvrevXba and Galvforcla2 which were tailed with *Xbal* and *Cla 1* restriction sites. The PCR products were then ligated into the plasmid FBMoSALF after *Xbal* and *Cla 1* digestion.

The chimaeric envelope expression plasmid EXGaLVMoT was constructed by PCR amplification of the cDNA encoding GALV env from plasmid pMOVGaLVSEATOenv using primers galvslq and galvforcla2. Primer "galvslq" was tailed with a *Notl* restriction site and contained the coding sequence for a factor Xa cleavage signal (IEGR). The PCR products were ligated into the plasmid EMo after *Notl* and *Cla1* digestion. The sequences of the primers are shown below. The restriction enzyme sites are underlined. The coding sequence for the factor Xa cleavage signal is shown in bold.

galvslq 5'gcaaatctgcggccgcaatcgagggaaggagtctgcaaaataagaacccccaccag 3'

galvforcla2 5'ccatcgattgatgcatggcccgag 3'

galvrevxba 5'ctagctctagaatggtattgctgcctgggtcc 3'

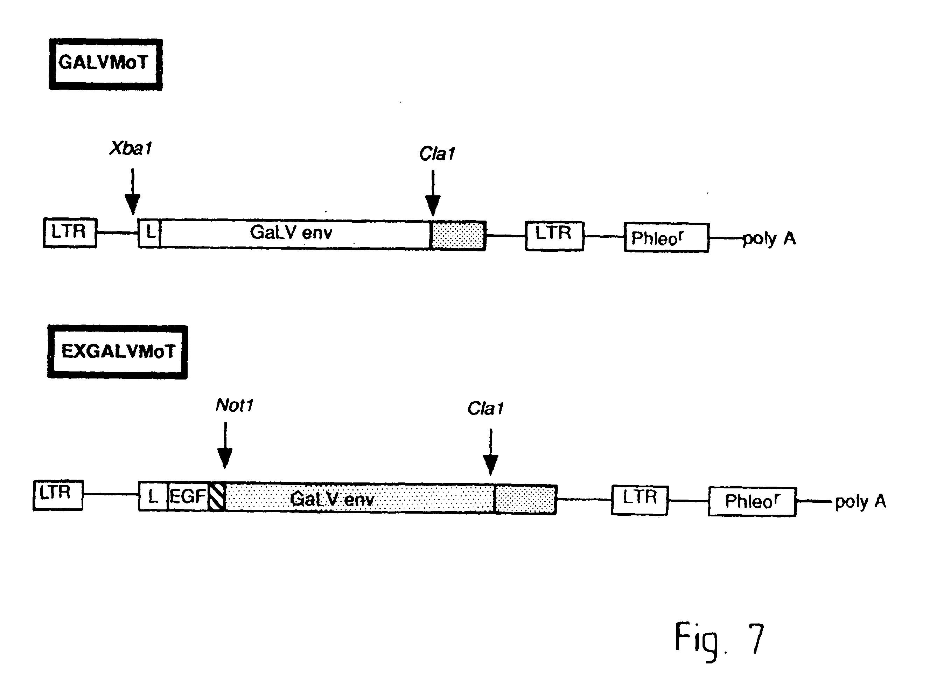

The correct sequence of both constructs was confirmed by didexoysequencing. A diagrammatic representation of the constructs is shown in figure 7.

### Vector production

The envelope expression plasmids were transfected into the TELCeB6 complementing cell line which contains gag-pol expression plasmid and an nls LacZ retroviral vector. Stable transfectants were selected in 50µg/ml phleomycin and pooled.

### Infection of target cells

Supernatant from the transfected TELCeB6 complementing cell lines was harvested after the cells had been allowed to grow to confluency at 37°C then placed at 32°C for 1-3 days. The medium was changed and, after overnight incubation, the supernatant was harvested and filtered through a 0.45µm filter. The filtered supernatants were then used to infect target cells. Adherent target cells were plated into six-well plates at approximately 10⁵ cells per well on the evening prior to infection and incubated overnight at 37°C and suspension cells were plated into six well plates at approximately 10⁶ cells per well one hour before infection. Filtered viral supernatant in serum free medium was added to the target cells and incubated for 2-4 hours in the presence of 8mg/ml polybrene. For infections involving factor Xa cleavage, the virus was incubated with 4mg/ml of factor Xa protease in the presence of 2.5mM CaCl₂ for 90 mins prior to infection. The retroviral supernatant was then removed from the target cells, the medium was replaced with the usual medium and the cells were placed at 37"C for a further 48-72 hours. X gal staining for detection of β-galactosidase activity was then carried out.

### Results

### Titration of GaLVMoT and EXGaLVMoT on HT1080 cells

When these vectors were titrated on HT1080 cells, a human EGF receptor positive cell line, the titre of GaLVMoT was 10⁶ efu/ml whereas that of EXGaLVMoT was 3.6 x10³ efu/ml. However, when the vector supernatant was incubated with factor Xa protease prior to infection, in order to cleave the displayed domain, the titre of GaLVMoT remained at 10⁶ efu/ml whereas the titre of EXGaLVMoT was increased to 3.6 x 10⁴/ml (table 4).

### Titration of GaLVMoT and EXGaLVMoT on MDBK cells

When these vectors were titrated on MDBK cells, a bovine EGF-R positive cell line, there was a similar finding. The titre of EXGaLVMoT was reduced compared to GaLVMoT but increased ten fold upon protease cleavage (table 4).

### Infection of haemopoietic cells with EXGaLVMoT

Two EGF-R negative haemopoietic suspension cell lines, HMC-1 and Meg-O1 were infected with EXGaLVMoT and gave titres (expressed a percentage blue cells) of 28.8% and 31.65% respectively. These results are similar to those previously published with the vector EXA (Fielding et at., Blood 91, 1-10, 1998). Taken in conjunction with the above data on the EGF-R positive cells, this suggests the EXGaLVMoT exhibits similar characteristics to the EXA vector where the displayed domain causes a reduction in infectivity in a receptor dependent manner.

**Table 4**

| Titre of GaLV vectors on EGF-R positive cells | | | | |
|---|---|---|---|---|
| | **HT1080** | | **MDBK** | |
| | **-Xa** | **+Xa** | **-Xa** | **+Xa** |
| GaLVMoT | 1x10⁶ | 1x10⁶ | 3.5x10⁴ | 2.9x104 |
| EXGaLVMoT | 3.6x10³ | 3.6x10⁴ | < 1 | 12 |

### Conclusions

1. Wild type (GaLVMoT) and chimaeric Gibbon Ape Leukaemia virus envelope expression constructs have been constructed and incorporated into retroviral vector particles which contain MLV gag-pol core particles and a Moloney MLV nlsLacZ retroviral vector.
2. Both the wild type and EGF-chimaeric vectors are capable of infecting human cell lines.
3. The titre of the EGF-chimaera is considerably reduced on EGF receptor positive cell lines and can be increased by factor Xa cleavage of the displayed domain. The largest reduction in titre is seen on cell lines with the highest density of EGF receptors.
4. Thus, display of EGF as an N terminal extension of the Gibbon Ape Leukaemia virus SU glycoprotein results in altered viral tropism which is similar to that seen with display of EGF on the murine leukaemia virus envelopes (Nilson et al., Gene Ther. 3, 280, 1996) and is likely to be EGF-receptor mediated.

## Claims

1. A pharmaceutical composition for use in gene therapy of a malignant disease comprising a recombinant nucleic acid vector in admixture with a pharmaceutically acceptable carrier, wherein the vector directs the expression on a eukaryotic cell surface of a fusogenic combination of separate paramyxoviral glycoproteins or a fusogenic membrane polypeptide which is other than a single paramyxoviral glycoprotein.

2. A pharmaceutical composition for use in gene therapy of a malignant disease comprising a combination of first and second recombinant nucleic acid vectors in admixture with a pharmaceutically acceptable carrier, wherein said first and second vectors, in combination, direct the expression on a eukaryotic cell surface of a fusogenic combination of separate paramyxoviral glycoproteins.

3. A pharmaceutical composition according to claim 1 or 2, wherein the vector or vector combination is present within a host cell.

4. A pharmaceutical composition according to claim 1, comprising a vector directing the expression on a eukaryotic cell surface of a measles virus H protein fused, at its C terminus, to another polypeptide.

5. A composition according to claim 1 or 2, wherein the vector directs the expression of at least a fusogenic portion of a viral fusogenic membrane glycoprotein (FMG).

6. A composition according to any one of claims 1, 2 or 3, wherein the vector directs the expression of a non-naturally occurring polypeptide.

7. A composition according to claim 1, 2 or 3, wherein the vector or vector combination directs the expression of a fusogenic membrane glycoprotein having an artificially introduced protease-sensitivity or binding specificity, or enhanced fusogenicity.

8. A composition according to claim 3, comprising a plurality of host cells, said host cells being selected from the group consisting of: neoplastic cells, migratory cells, T lymphocytes, B lymphocytes or other haemopoietic cells.

9. Use of a recombinant nucleic acid vector in the preparation of a medicament to treat a malignant disease in a human patient, wherein the medicament comprises a composition in accordance with any one of the preceding claims.

10. A use according to claim 9, wherein the vector is present in a host cell.

## Patentansprüche

1. Pharmazeutische Zusammensetzung zur Verwendung in der Gentherapie einer bösartigen Erkrankung, umfassend einen rekombinanten Nukleinsäurevektor in Beimischung mit einem pharmazeutisch verträglichen Träger, wobei der Vektor die Expression auf einer eukaryotischen Zelloberfläche einer fusogenen Kombination von getrennten paramyxoviralen Glykoproteinen oder eines fusogenen Membranpolypeptids, das von einem einzelnen paramyxoviralen Glykoprotein verschieden ist, steuert.

2. Pharmazeutische Zusammensetzung zur Verwendung in der Gentherapie einer bösartigen Erkrankung, umfassend eine Kombination aus ersten und zweiten rekombinanten Nukleinsäurevektoren in Beimischung mit einem pharmazeutisch verträglichen Träger, wobei die ersten und zweiten Vektoren zusammen die Expression auf einer eukaryotischen Zelloberfläche einer fusogenen Kombination von getrennten paramyxoviralen Glykoproteinen steuern.

3. Pharmazeutische Zusammensetzung gemäß Anspruch 1 oder 2, wobei der Vektor oder die Vektorkombination innerhalb einer Wirtszelle vorliegt.

4. Pharmazeutische Zusammensetzung gemäß Anspruch 1, umfassend einen Vektor, der die Expression auf einer eukaryoten Zelloberfläche eines Masern Virus H Proteins, das an seinem C Terminus mit einem anderen Polypeptid fusioniert ist, steuert.

5. Zusammensetzung gemäß Anspruch 1 oder 2, wobei der Vektor die Expression von wenigstens einem fusogenen Anteil eines viralen fusogenen Membranglykoproteins (FMG) steuert.

6. Zusammensetzung gemäß einem der Ansprüche 1, 2 oder 3, wobei der Vektor die Expression eines nicht natürlicherweise vorkommenden Polypeptids steuert.

7. Zusammensetzung gemäß Anspruch 1, 2 oder 3, wobei der Vektor oder die Vektorkombination die Expression eines fusogenen Membranglykoproteins steuert, die eine künstlich eingeführte Protease-Sensitivität oder Bindungsspezifität oder erhöhte Fusogenität aufweist.

8. Zusammensetzung gemäß Anspruch 3, umfassend eine Vielzahl von Wirtszellen, die Wirtszellen sind ausgewählt aus der Gruppe bestehend aus: neoplastischen Zellen, wandernden Zellen, T Lymphozyten, B Lymphozyten und anderen hämatopoetischen Zellen.

9. Verwendung eines rekombinanten Nukleinsäurevektors in der Herstellung eines Medikaments, um eine bösartige Erkrankung in einem humanen Patienten zu behandeln, wobei das Medikament eine Zusammensetzung gemäß einem der vorhergehenden Ansprüche aufweist.

10. Verwendung gemäß Anspruch 9, wobei der Vektor in einer Wirtszelle anwesend ist.

## Revendications

1. Composition pharmaceutique pour une utilisation dans une thérapie génique d'une maladie maligne comprenant un vecteur d'acide nucléique recombinant en mélange avec un support pharmaceutiquement acceptable, dans laquelle le vecteur contrôle l'expression à la surface d'une cellule eucaryote d'une combinaison fusogénique de glycoprotéines paramyxovirales séparées ou d'un polypeptide membranaire fusogénique qui est autre qu'une glycoprotéine paramyxovirale seule.

2. Composition pharmaceutique pour une utilisation dans une thérapie génique d'une maladie maligne comprenant une combinaison d'un premier et d'un second vecteurs d'acide nucléique recombinants en mélange avec un support pharmaceutiquement acceptable, dans laquelle lesdits premier et second vecteurs, en combinaison, contrôlent l'expression à la surface d'une cellule eucaryote d'une combinaison fusogénique de glycoprotéines paramyxovirales séparées.

3. Composition pharmaceutique selon la revendication 1 ou 2, dans laquelle le vecteur ou la combinaison de vecteurs est présent(e) dans une cellule hôte.

4. Composition pharmaceutique selon la revendication 1, comprenant un vecteur contrôlant l'expression à la surface d'une cellule eucaryote de la protéine H d'un virus de la rougeole fusionnée, à son extrémité C terminale, à un autre polypeptide.

5. Composition pharmaceutique selon la revendication 1 ou 2, dans laquelle le vecteur contrôle l'expression d'au moins une partie fusogénique d'une glycoproteine membranaire fusogénique virale (FMG).

6. Composition selon l'une quelconque des revendications 1, 2 ou 3, dans laquelle le vecteur contrôle l'expression d'un polypeptide n'existant pas naturellement.

7. Composition selon la revendication 1, 2 ou 3, dans laquelle le vecteur ou la combinaison de vecteurs contrôle l'expression d'une glycoprotéine membranaire fusogénique ayant une sensibilité à une protéase, une spécificité de liaison ou une fusogénicité augmentée, artificiellement introduite.

8. Composition selon la revendication 3, comprenant une pluralité de cellules hôtes, lesdites cellules hôtes étant sélectionnées parmi le groupe consistant en : des cellules néoplasiques, des cellules migratrices, des lymphocytes T, des lymphocytes B ou d'autres cellules hématopoïétiques.

9. Utilisation d'un vecteur d'acide nucléique recombinant pour la préparation d'un médicament destiné à traiter une maladie maligne chez un patient humain, dans laquelle le médicament comprend une composition selon l'une quelconque des revendications précédentes.

10. Utilisation selon la revendication 9, dans laquelle le vecteur est présent dans une cellule hôte.
